# EUROPÄISCHE PATENTSCHRIFT

(11) **EP 2 338 844 B1**
(45) Veröffentlichungstag und Bekanntmachung des Hinweises auf die Patenterteilung: **30.07.2014**
(21) Anmeldenummer: 10195713.2
(22) Anmeldetag: 17.12.2010
(51) Int. Cl.: C02F 3/00, C02F 3/06, C02F 3/10, C02F 3/12, C12N 11/08, C12N 11/14

(54) **Verfahren zur biologischen Aufbereitung von Reinwasser aus Badegewässern und Schwimmteich oder biologisch aufbereiteter Pool**
Method of biologically processing pre-treated bathing water, and a swimming pond or biologically treated pool
Procédé pour le traitement biologique d'eau prétraitée provenant d'eaux de baignade, et piscine naturelle ou étang traité biologiquement

(30) Priorität: 23.12.2009 AT 20372009
(43) Veröffentlichungstag der Anmeldung: 29.06.2011
(73) Patentinhaber: Wesner, Wolfgang, 1050 Wien (AT); Kurzmann, Heimo, 5302 Henndorf (AT)
(72) Erfinder: Wesner, Herr Wolfgang, 1050 Wien (AT); Kurzmann, Herr Heino, 5302 Henndorf (AT)
(74) Vertreter: Vinazzer, Edith

(56) Entgegenhaltungen:
- EP-A2- 0 607 636
- DE-A1- 19 807 406
- DE-A1- 19 813 022
- US-A1- 2004 018 609
- DATABASE WPI Week 200547 Thomson Scientific, London, GB; AN 2005-464188 XP002634235, -& KR 2005 018 777 A (LEE J M) 28. Februar 2005 (2005-02-28)
- DATABASE WPI Week 199719 Thomson Scientific, London, GB; AN 1997-207493 XP002634232, -& JP 9 057291 A (NIPPON TOKUSHU PIPE HANBAI KK) 4. März 1997 (1997-03-04)
- DATABASE WPI Week 198913 Thomson Scientific, London, GB; AN 1989-098061 XP002634233, -& JP 1 047424 A (NICHIAS CORP) 21. Februar 1989 (1989-02-21)
- DATABASE WPI Week 200915 Thomson Scientific, London, GB; AN 2009-B41831 XP002634234, -& KR 100 836 661 B1 (UNIV KOREA IND & ACADEMIC COLLABORATION) 10. Juni 2008 (2008-06-10)

## Beschreibung

Die Erfindung betrifft ein *Verfahren zur biologischen Aufbereitung von Reinwasser - Wasser, welches bereits biologisch und*/*oder chemisch*/*physikalisch aufbereitet wurde - aus Badegewässern, wobei das Reinwasser einen* Schwebebettfilter mit einem *Filtersubstrat durchströmt, welches aus künstlich hergestellten Substratteilchen besteht. Die Erfindung betrifft ferner einen Schwimmteich oder einen biologisch aufbereiteter Pool mit einem* Schwebebettfilter *enthaltend ein Filtersubstrat, mit welchem Reinwasser - Wasser, welches bereits biologisch und*/*oder chemisch*/*physikalisch aufbereitet wurde - aus den Badegewässern aufbereitet wird, und welches aus künstlich hergestellten Substratteilchen besteht.*

Es sind passive bzw. inerte Filtermedien bekannt, welche eine große angeströmte Fläche für die Ausbildung von Biofilmen anbieten. Zu den passiven Filtermaterialen zählen Kies, Blähton, Keramikteilchen, Steinwolle oder Kunststoffkörper. Darüber hinaus sind aktive filtermedien bekannt, welche an der vom Wasser angeströmten Oberfläche lokal Nährstoffe anbieten, woraus eine höhere Biomassebildung resultiert.

*Die* EP 0 607 636 A2 *offenbart ein Filtersubstrat mit Substratteilchen, welche unter anderem aus Aktivkohle und Zeolith Hergestellt werden. Die speziell definierten Poren der Substratteilchen dienen als Oberflächen zur Ansiedlung von Bakterien oder sonstigen Mikroorganismen. Die in diesem Dokument erwähnten Zeolithe dürften, wie alle natürlich vorkommenden Zeolithe, phosphathaltig sein.*

*Die* US 2004/018609 A1*, beschreibt ein Trägermaterial für Biofilme, welches Nährstoffe für einen Biofilm zur verfügung stellt, mit dem Ziel, einen Biofilm zur Produktion von biochemischen Produkten zu versorgen. Als alternativer Anwendungszweck ist die Reinigung von Abwasser angegeben.*

*Aus der* KR 2005 018777 A *ist ein Tragermaterial zur Abwasseraufbereitung bekannt, welches natürlichen Zeolith enthält. Die* JP 9 057291 A *befasst sich mit der Herstellung von Trägerkugeln mit Poren und großen Oberflächen, insbesondere aus Glasschaum.*

*Die* DE 198 07 406 A1 *offenbart ein Filtersubstrat und dessen Herstellung aus gemahlenem Naturzeolith. Naturzeolith ist reich an Nährstoffen und für die Abwasserreinigung, aber nicht fur die Reinwasseraufbereitung geeignet. Die* JP 1 047424 A *beschreibt die Verwendung von Zeolith zur Geruchsbekämpfung in Luft. Die* ZA 200 801 824 A *befasst sich mit der Denitrifikation mit Schwefel unter Lichteinwirkung. Aus der* DE 198 13 022 A1 *ist ein Trägermaterial aus einem Biopolymer bekannt, welches langsam Nährstoffe freisetzen kann.* WO 2009/141195 beschreibt ein Verfahren zur biologischen Aufbereitung von Badewasser unter Verwendung eines biologischen Filters mit einen auf einem Trägersubstrat gewachsenen Biofilm. Die biologische Aufbereitung von Reinwasser mit passiven Filtermaterialien ist meist ineffektiv, da Reinwasser, welches ja bereits eine biologische und/oder chemischphysikalische Wasseraufbereitung durchlaufen hat, ein unausgewogenes Nahrstoftverhältnis aufweist.

Das als Minimumgesetz von Justus von Liebig bekannte Modell besagt, dass das Wachstum von Pflanzen durch die im Verhältnis knappste Ressource (Nährstoffe, Wasser, Licht etc.) eingeschränkt wird. Diese Ressource wird auch als Minimumfaktor bezeichnet. Ein Faktor hat keinen Einfluss auf das Wachstum, wenn eine Ressource hinzugegeben wird, die bereits im benötigten Umfang vorhanden ist. Im übertragenen Sinn kann dieses Gesetzt auf Biofilm angewandt werden. Bei Biofilm, welcher kein Licht benötigt und mit Wasser stets ausreichend versorgt ist, ist der Minimumfaktor üblicher Weise der Mangel eines Elementes wie Stickstoff, Phosphor, Kohlenstoff, etc.. Um eine Wachstumseinschränkung des Biofilms aufzuheben, muss dem Biofilm daher das betreffende Mangelelement zugeführt werden.

Das Redfield-Verhältnis beschreibt die Anteile der atomaren Zusammensetzung von maritimem Phytoplankton und beträgt 1 Mol P : 16 Mol N : 106 Mol C. Ähnliche Verhältniszahlen wurden für Biofilme definiert, wobei für eine vollständige Darstellung weitere chemische Elemente einbezogen werden. Zentrale Einheit der klassischen biologischen Aufbereitung in Kläranlagen stellt das Belebtschlammbecken dar. Schwebende Biofilmaggregate (Flocke) werden gebildet, wobei die im Wasser gelösten Nährstoffe gebunden werden. Die biologische Aufbereitung kommt beim Erreichen der Limitierung des ersten essentiellen Nährstoffelements zum Stillstand. Oft ist jedoch hier das gewünschte Aufbereitungsziel noch nicht erreicht, dieses kann erst durch einen weiteren Abbau unter Zugabe dieses Mangelelementes erfolgen.

Je nach der "Herkunft" und dem Verwendungszweck des Reinwassers sind unterschiedliche Qualitätskriterien wesentlich bzw. erwünscht. Die gängigen Qualitätskriterien von Reinwasser betreffen den Gehalt an Kohlenstoffverbindungen, die Oxidierbarkeit ("Permanganatverbrauch") oder die Sauerstoffzehrung (CSB bzw. Chemischer Sauerstoffbedarf, BSB5 bzw. Biologischer Sauerstoffbedarf in 5 Tagen), den Gehalt an Stickstoff oder den Phosphatgehalt. Reinwasser weist üblicherweise nur eines dieser Kriterien auf, ist demnach entweder "kohlenstofflimitiert", "stickstofflimitiert" oder "phosphorlimitiert", das heißt, es enthält kaum/keine Kohlenstoffverbindungen, Stickstoffverbindungen oder Phosphorverbindungen. Soll eine Limitierung eines anderen "Elements" erreicht werden, werden nach dem Stand der Technik bekannte chemischphysikalische Methoden, wie Phosphatfällung mit Eisen-, Aluminium- oder Kalziumsalzen, oder Denitrifikationsstufen zur Stickstoffehminierung, eingesetzt.

Aktive Filtermaterialen ermöglichen durch das lokale Angebot von Mangelnährstoffen den Aufbau von Biomasse/ Biofilm aus den im Reinwasser verbliebenen Reststoffen, wodurch eine Weiterführende Wasserreinigung mit gezielter Reduzierung mehrerer Elemente gleichzeitig, unabhängig von der Ausgangszusammensetzung der Nährelemente des aufzubereitenden Wassers, erreicht wird.

So ist beispielsweise für die biologische Aufbereitung von Badewässern eine Phosphorlimitierung erwünscht. Eine Phosphorlimitierung auf unter 10 µg/l P verhindert das Algenwachstum und eine Limitierung auf unter 4 µg/l P reduzierst drastisch das Biofilmwachstum an der Beckenwand trotz Wasseranströmung. Reinwasser, welches aus der Aufbereitung des Badewassers im Kreislauf mit einem passiven Filter, beispielsweise einem Kiesfilter, stammt, wird durch die biofilmgebundene Denitrifikation an der Kiesoberfläche stickstofflimitiert, die erwünschte Phosphorlimitierung wird meist nicht erreicht.

Wird ein aktives Filtermaterial verwendet, welches in der Lage ist, lokal an seiner Oberfläche Stickstoff anzubieten, so können große Mengen an Phosphor und Kohlenstoff im neu aufgebauten Biofilm festgelegt werden, ohne dass es zu einer Stickstoffanreicherung im Wasser kommt. Zu den in der Wasseraufbreitung bekannten Naturstoffen, welche in der Lage sind, diese Aufgabe zu erfüllen, zählen Zeolithe, Gerstenstroh und Erlenzapfen. Zeolithe finden universale Anwendung in der Reinigung von Abwässern aus der Industrie, Wasser aus Schwimmteichen, sonstigen Badewässern und Aquarien. An der Oberfläche von natürlichen Zeolithen erfolgt insbesondere eine ionische Ammonium- und Kaliumbindung. Gerstenstroh eignet sich für die Aufbereitung von Wasser aus Schwimmteichen, wo ein Phosphormangel erwünscht ist, Erlenzapfen zur Aufbereiten von Aquariumwasser mit dem Ziel der Phosphorlimitierung.

Die Entwicklung neuer künstlicher Filtermedien ist deshalb von großem Interesse, weil Naturstoffe in gleichbleibender Qualität und in chemisch reiner Form nicht verfügbar sind. So weist etwa natürlicher Zeolith oft einen hohen Phosphatgehalt auf, welcher diesen Zeolith für ein Filtermaterial zur Bindung von Phosphaten ungeeignet macht. Die Güte eines Filtersubstrates zur biologischen Bindung von Phosphaten ergibt sich aus dem Verhältnis der in Mangel befindlichen Elemente zu dem zu fixierenden Element. So gilt für Naturzeolithe: Ein geeignetes Substrat zur biologischen Phosphatfixierung für Badegewässer weist ein Stickstoff : Phosphor-Verhältnis>10:1 auf. Ein optimales Filtersubstrat sollte jedoch überhaupt keinen verfügbaren Phosphor enthalten.

Selbst wenn das Stickstoff : Phosphor-Verhältnis >10:1 beträgt, aber Phosphor im Filtersubstrat enthalten ist, so kann es bei Störungen in der gleichmäßigen Anströmung (etwa durch Pumpenausfall, durch verminderte Pumpenleistung oder einer sonstigen ungleichmäßige Durchströmung des Filtersubstrates) zu einer massiven Phosphorfreisetzung als Folgte von lokalem Sauerstoffmangel kommen.

Der Erfindung liegt die Aufgabe zugrunde, *ein Verfahren und einen Schwimmteich oder biologisch aufbereiteten Pool zur Verfügung zu stellen, welche eine weiterführende Aufbereitung von Reinwasser aus Badegewässern durch optimiertes Biofilmwachstum ermöglichen.*

Gelöst wird die gestellte Aufgabe erfindungsgemäß dadurch, *dass die Substratteilchen in Wasser schwimmende Trägerteilchen aufweisen, welche mit einer Ummantelung versehen sind, die künstlichen phosphatfreien Zeolith enthält, wobei die Substratteilchen in einer Nährlösung durch Ionenaustausch derart konditioniert worden sind, dass die von der Synthese des Zeoliths stammenden Natriumionen durch Ammonium-, Kalium- und Magnesiumionen ersetzt sind.*

*Gemäß der Erfindung wird ein Filtersubstrat zur Verfügung gestellt welches keinen biologisch verfügbaren Phosphor enthält, was bei Naturmaterialien nicht der Fall ist.* Das Filtersubstrat gemäß der Erfindung ersetzt die bekannten, oben erwähnten chemischphysikalischen Methoden zur Limitierung eines Elements.

Es gibt eine Vielzahl von Möglichkeiten, Substraneilchen gemäß der Erfindung auszufuhren bzw. herzustellen. Bei einer besonders vorteilhaften Ausführungsform der Erfindung bestehen die Trägerteilchen aus Glasschaum, Ton, Kies, Gesteinsbruch, Kunststoff und die Ummantelung besteht aus Zeolithpulver oder aus Mischungen von Zeolithpulver mit zumindest einem Bindemittel. Derartige Substratteilchen können ein geringeres spezifisches Gewicht als Wasser aufweisen und sind daher in einem Schwebebettfilter optimal einsetzbar.

Als Bindemittel eignen sich anorganische Bindemittel, beispielsweise Zement, SiO₂ oder Kalk, oder organische Bindemittel, beispielsweise wasserbeständiger Klebstoff oder ein Polymer, wie Phenolharz oder ein Acrylat.

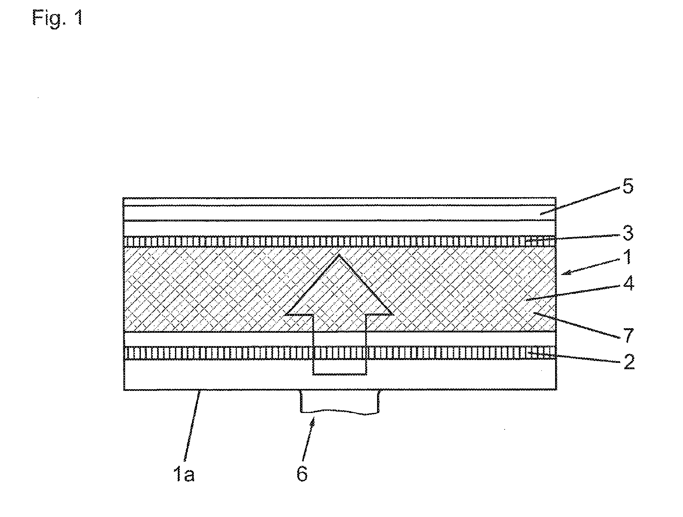

Weitere Merkmale. Vorteile und Einzelheiten der Erfindung werden nun anhand der schematischen Zeichnung näher erläutert. Dabei zeigt die einzige Figur, Fig. 1, eine Ansicht eines Filters mit einem Filtersubstrat gemäß der Erfindung.

In der Beschreibung und den Patentansprüchen sind unter den Begriffen "Element", "limitierendes Element", "Mangelelement" und "Nährstoff" alle das jeweilige chemische Element enthaltenden Salze, Ionen und neutrale Verbindungen zu verstehen, beispielsweise im Fall von Phosphor insbesondere alle Phosphate und organische Phosphorverbindungen, im Fall von Stickstoff insbesondere Nitrate, Nitrite und Ammonium, und organische Stickstoffverbindungen, im Fall von Kohlenstoff insbesondere alle organischen Kohlenstoffverbindungen.

Die Erfindung befasst sich mit einem Filtersubstrat zur biologischen Aufbereitung von Reinwasser. Unter dem Begriff Reinwasser wird Wasser verstanden, welches bereits einer biologischen und/oder chemisch-physikalischen Wasseraufbereitung unterzogen worden ist. *Das Reinwasser stammt von Badegewässern, insbesondere aus Swimmingpools oder Schwimmteichen, und gilt in Folgte Filtration, Flockung, Desinfektion oder dergleichen oder auch nur auf Grund der Kreislaufführung über immer denselben Filter als Reinwasser.*

Der in Fig. 1 beispielhaft gezeigte Filter ist ein Schwebebettfilter und weist einen beispielsweise quaderförmigen oder zylindrischen Behälter 1 auf, welcher oben offen ist und an seinem Boden 1a zumindest einen Zufluss 6 für das zu filternde Reinwasser aufweist, sodass der Behälter 1 von unten nach oben von Wasser durchströmt wird. Der Zufluss kann auch als Abfluss genutzt werden und die Durchströmung kann von oben nach unten erfolgen. Alternativ ist eine horizontale Durchströmung über geeignete Ein- und Auslässe möglich, An der Innenwand des Behälters 1 sind parallel zum Boden des Behälters 1 zwei Siebplatten 2 und 3 unter einem gegenseitigen Abstand gehalten, beispielsweise eingehängt, zwischen welchen ein Filtersubstrat 4 eingebracht ist. Ist der Behälter 1 mit Reinwasser gefüllt, schwimmt Filtersubstrat 4, welches ein geringeres spezifisches Gewicht als Wasser aufweist, auf, wird gegen die Siebplatte 2 gedrückt und bildet eine stabile Filterschüttung. Alternativ kann das Filtermaterial auch schwerer als Wasser sein und im Betrieb auf der unteren Platte 3 liegen. Das im Filtersubstrat 4 aufbereitete Wasser gelangt in eine Überlaufrinne 5 am oberen Rand des Behälters 1 und kann von dort beispielsweise unmittelbar einem Schwimmbecken, einem Schwimmteich, einer weiteren biologischen Filtereinheit oder einem Pflanzenbecken zugeführt werden. Eine Reinigung des Behälters 1 und der Siebplatte 2, 3 oder ein Tausch des Filtersubstrates 4 ist auf einfache Weise nach Ablassen des Wassers möglich.

Das Filtersubstrat 4 gemäß der Erfindung besteht aus Substratteilchen 7, welche künstlich hergestellt sind und derart konditioniert sind, dass sie die für die Aufbereitung *des Reinwassers* benötigten Nährstoffe bzw. Mangelelemente an ihrer Oberfläche zur Verfügung stellen, sodass die Aufbereitung des Reinwassers durch Biofilmwachstum ermöglicht ist. Die Substratteilchen 7 gemäß der Erfindung sind *ferner derart* ausgeführt bzw. hergestellt, dass sie jene Elemente bzw. jenes Element, welche bzw. welches *auf die Anwendung* abgestimmt das/die zu limitierende(n) Element(e) im aufbreiteten Reinwasser werden soll bzw. sollen nicht in biologisch verfügbarerer Form enthalten. Zu den Mangetelementen für den Biofilmaufbau aus Reinwasser gehören *insbesondere Stickstoff,* Kohlenstoff, Phosphor, Kalium, Magnesium, Kalzium, Mangan und Eisen. Das zu limitierende Element in Reinwasser von Schwimmteichen, Swimmingpools und Badegewässern *ist Phasphor.*

Substratteilchen 7 gemäß der Erfindung können auf unterschiedliche Weise aufgebaut sein und hergestellt werden. Die Größe der Substratteilchen kann jener bekannter Filtersubstratteilchen entsprechen.

Bei einer erfindungsgemäßen Ausfuhrungsform wird künstlich hergestellter (synthetisierter) Zeolith in Pulverform als Ausgangsmaterial verwendet. Das Zeolithpulver wird zu Pellets oder sonstigen Formkörpern gepresst, wobei Zeolithpulver mit einem Bindemittel wie Zement, Wasserglas, Brandkalk, anorganischen oder organischen Polymerbindern bzw. Harzen und/oder Füllstoffen wie Kalziumkarbonat, Gesteinsmehlen, Tonen, oder organischen oder anorganischen Polymeren vermischt wird, um die Dichte der Ladungen an der Oberfläche der hergestellten Substratteilchen herabzusetzen. Bei einer bevorzugten Ausführungsform wird Zeolithpulver mit feuchtem, ungebrannten Ton vermischt, die hergestellte Mischung pelletiert und die Pellets gebrannt. Je nach Restfeuchte bläht sich das Material beim Brennen mehr oder weniger auf.

Bei einer weiteren Ausführungsform bestehen die Substratteilchen 7 aus einem Trägermaterial mit einer Ummantelung, die synthetisierten Zeolith enthält. Als Trägermaterial eignen sich beispielsweise Glasschaum, gebrannter Ton, Kies oder Bruch diverser Gesteine. Die Ummantelung wird aus Zeolithpulver durch Bestäuben erzeugt oder aus einer Mischung aus Zeolithpulver mit zumindest einem Bindemittel, welches organisch oder anorganisch sein kann, hergestellt. Als anorganische Bindemittel kommen beispielsweise Zement, SiO₂ (Wasserglas) oder Kalk in Frage. Als organische Bindemittel eignen sich diverse wasserbeständige Klebstoffe oder Polymere, wie Phenolharze oder Acrylate.

Zeolith ist bekannter Weise ein Kationenaustauscher und es wird die Ionentauscher-Funktion von Zeolith genützt. Die fertig hergestellten und zumindest außenseitig, etwa in der erwähnten Ummantelung, Zeolith beinhaltenden Substratteilchen 7 werden nach ihrer Herstellung mit *den benötigten* Nährstoffionen beladen bzw. konditioniert. Eine biologische Voraktivierung, bei der direkt bei der Produktion biofilmbildende Bakterien durch Sprühen, oder Einschwemmen aufgebracht werden bzw. wo bereits ein erster Biofilm auf den Filtersubstratteilchen 7 angezüchtet wird, ist möglich. Die im künstlichen Zeolith üblicher Weise von der Produktion stammenden Natriumionen können in geeigneten Salzlösungen gegen andere Kationen, beispielsweise Ammonium-, Kalium- oder Magnesiumkationen getauscht werden.

Nachstehend wird ein Beispiel für ein Filtersubstrat ausführlicher beschrieben.

### Beispiel:

### Filtersubstrat für Schwimmteiche und biologisch aufbereitete Pools

In Schwimmteichen oder biologisch aufbereiteten Swimmingpools steht die Phosphorlimitierung im Vordergrund, um das Algenwachstum in allen Bereichen der Anlage und das Biofilmwachstum im Schwimmbereich, insbesondere an der Beckenwand, zu vermeiden. Die Kohlenstoffversorgung des Badewassers ist durch die Einträge der Badegäste im Allgemeinen ausreichend, die Versorgung mit Stickstoff ist meist in Folge der Stickstoffzehrung durch Denitrifikationsprozesse in Mangel. Es ist daher günstig, Stickstoff künstlich über das Filtersubstrat gemäß der Erfindung zuzuführen. Damit die Elemente Kalium und Magnesium sowie andere Spurenelemente dadurch nicht in Mangel geraten, sollten auch diese über das Filtersubstrat angeboten werden. Ein für diesen Einsatzzweck geeignetes, künstlich hergestelltes Filtersubstrat soll daher die Kationen Stickstoff, Kalium und Magnesium sowie Spurenelemente an seiner Oberfläche biologisch verfügbar anbieten. Ausgangsmaterial für das Filtersubstrat sind vorzugsweise in Wasser schwimmende Trägerteilchen aus Glasschaum, welche mit einer Ummantelung versehen sind, die künstlichen phosphatfreien Zeolith enthält. Alternativ dazu können Substratteilchen aus durch Ätzen aktivierter Steinwolle eingesetzt werden. Um die erwünschten Ionen an der Oberfläche des Filtersubstrats anzubringen, wird dieses in einer entsprechenden Nährlösung durch Ionenaustausch konditioniert. Dabei werden die ursprünglich von der Synthese des Zeoliths stammenden Natriumionen durch Ammonium-, Kalium- und Magnesiumionen ersetzt. Dieses Substrat wird beispielsweise in einem Schwebebettfilter, wie in Fig. 1 dargestellt, gleichmäßig angeströmt, sodass ein Biofilmaufbau aus den am Filtermaterial gespeicherten Elementen Stickstoff, Kalium und Magnesium mit den Phosphaten und den gelösten Kohlenstoffverbindungen aus dem angeströmten Wasser möglich ist. Der Biofilm am Filtersubstrat kann geerntet werden, indem die Wasserzufuhr unterbrochen wird und der Filter eine Woche eingestaut wird. Dadurch wird die Sauerstoffanlieferung unterbunden, wodurch sich der Biofilm verflüssigt und die Nährstoffe (inklusive der Phosphate) in Lösung gehen. Diese Lösung wird nach einer Woche abgepumpt und verworfen, wodurch die Phosphate aus dem Kreislauf des Beckenwassers endgültig entfernt werden. Nach Spülung der Filtermaterials mit Frischwasser können neue Nährstoffionen aus einer Lösung, mit der das Filtersubstrat gespült wird, an der Oberfläche des Filtermaterials gespeichert werden, wodurch das Substrat regeneriert wird und die ursprüngliche Nährstoffkonfiguration wieder hergestellt wird.

### BEZUGSZIFFERNLISTE

- 1: Behälter
- 1a: Boden
- 2: Siebplatte
- 3: Siebplatte
- 4: Filtersubstrat
- 5: Überlaufrinne
- 6: Zufluss
- 7: Substratteilchen

## Patentansprüche

1. Verfahren zur biologischen Aufbereitung von Reinwasser - Wasser, welches bereits biologisch und/oder chemisch/physikalisch aufbereitet wurde -- aus Badegewässern, wobei das Reinwasser einen Schwebebettfilter mit einem Filtersubstrat durchströmt, welches aus künstlich hergestellten Substratteilchen besteht,
**dadurch gekennzeichnet,**
**dass** die Substratteilchen in Wasser schwimmende Trägerteilchen aufweisen, welche mit einer Ummantelung versehen sind, die künstlichen phosphatfreien Zeolith enthält, wobei die Substratteilchen in einer Nährlösung durch Ionenaustausch derart konditioniert worden sind, dass die von der Synthese des Zeoliths stammenden Natriumionen durch Ammonium-, Kalium- und Magnesiumionen ersetzt sind.

2. Verfahren nach Anspruch 1, **dadurch gekennzeichnet, dass** die Trägerteilchen aus Glasschaum, Ton, Kies, Gesteinsbruch oder Kunststoff bestehen und die Ummantelung aus Zeolithpulver oder aus Mischungen von Zeolithpulver mit zumindest einem Bindemittel besteht.

3. Verfahren nach Anspruch 2, **dadurch gekennzeichnet, dass** das Bindemittel ein anorganisches Bindemittel, beispielsweise Zement, SiO₂ oder Kalk, oder ein organisches Bindemittel, beispielsweise ein wasserbeständiger Klebstoff oder ein Polymer, wie ein Phenolharz oder ein Acrylat, ist.

4. *Schwimmteich oder biologisch aufbereiteter Pool mit einem* Schwebebettfilter *enthaltend ein* Filtersubstrat, mit welchem Reinwasser - Wasser, welches bereits biologisch und/oder chemisch/physikalisch aufbereitet wurde - *aus den* Badegewässern aufbereitet wird, und welches aus künstlich hergestellten Substratteilchen besteht,
**dadurch gekennzeichnet,**
die die Substratteilchen in Wasser schwimmende Trägerteilchen aufweisen, welche mit einer Ummantelung versehen sind, die künstlichen phosphatfreien Zeolith enthält, wobei die Substratteilchen in einer Nährlösung durch Ionenaustausch derart konditioniert worden sind, dass die von der Synthese des Zeoliths stammenden Natriumionen durch Ammonium-, Kalium- und Magnesiumionen ersetzt sind.

5. *Schwimmteich oder biologisch aufbereiteter Pool* nach Anspruch 4, **dadurch gekennzeichnet, dass** *die Trägerteilchen der Substratteilchen* aus Glasschaum, Ton, Kies, Gesteinsbruch oder Kunststoff bestehen und die Ummantelung aus Zeolithpulver oder aus Mischungen von Zeolithpulver mit zumindest einem Bindemittel besteht.

6. *Schwimmteich oder biologisch aufbereiteter Pool* nach Anspruch 5, **dadurch gekennzeichnet, dass** das Bindemittel ein anorganisches Bindemittel, beispielsweise Zement. SiO₂ oder Kalk, oder ein organisches Bindemittel, beispielsweise ein wasserbeständiger Klebstoff oder ein Polymer, wie ein Phenolharz oder ein Acrylat, ist

## Claims

1. A method for biologically preparing pure water - water that has already been biologically and/or chemically/physically prepared - from bathing water, wherein the pure water flows through a fluidized bed filter with a filter substrate, which consists of artificially produced substrate particles,
**characterised in that**
the substrate particles have carrier particles floating in water, which are provided with a jacket that contains artificial phosphate-free zeolite, wherein the substrate particles have been conditioned in a nutrient solution by means of ion exchange in such a manner that the sodium ions stemming from the synthesis of the zeolite are replaced by ammonium, potassium and magnesium ions.

2. The method according to Claim 1, **characterised in that** the carrier particles consist of foam glass, clay, gravel, crushed rocks or plastic and the jacket consists of zeolite powder or of mixtures of zeolite powder with at least one binding agent.

3. The method according to Claim 2, **characterised in that** the binding agent is an inorganic binding agent, for example cement, SiO₂ or chalk, or an organic binding agent, for example a water-resistant adhesive or a polymer, such as a phenolic resin or an acrylate.

4. A swimming pond or biologically prepared pool with a fluidized bed filter containing a filter substrate, using which pure water - water that has already been biologically and/or chemically/physically prepared - is prepared from the bathing water, and which consists of artificially produced substrate particles, **characterised in that**
the substrate particles have carrier particles floating in water, which are provided with a jacket that contains artificial phosphate-free zeolite, wherein the substrate particles have been conditioned in a nutrient solution by means of ion exchange in such a manner that the sodium ions stemming from the synthesis of the zeolite are replaced by ammonium, potassium and magnesium ions.

5. The swimming pond or biologically prepared pool according to Claim 4, **characterised in that** the carrier particles of the substrate particles consist of foam glass, clay, gravel, crushed rocks or plastic and the jacket consists of zeolite powder or of mixtures of zeolite powder with at least one binding agent.

6. The swimming pond or biologically prepared pool according to Claim 5, **characterised in that** the binding agent is an inorganic binding agent, for example cement, SiO₂ or chalk, or an organic binding agent, for example a water-resistant adhesive or a polymer, such as a phenolic resin or an acrylate.

## Revendications

1. Procédé destiné au traitement biologique d'eau pure (eau qui a déjà subi un traitement biologique et/ou chimique/physique) d'eaux de baignade, l'eau pure circulant à travers un filtre à lit flottant avec un substrat de filtrage qui est constitué de particules de substrat produites artificiellement,
**caractérisé en ce que**
les particules de substrat comportent des particules porteuses qui sont dotées d'une enveloppe qui contient de la zéolithe synthétique sans phosphate, les particules de substrat ayant été conditionnées dans une solution nutritive par échange d'ions de telle sorte que les ions de sodium issus de la synthèse de la zéolithe soient remplacés par des ions d'ammonium, des ions de potassium et des ions de magnésium.

2. Procédé selon la revendication 1, **caractérisé en ce que** les particules porteuses sont constituées de mousse de verre, d'argile, de graviers, de débris de pierre ou de matière plastique et **en ce que** l'enveloppe est constituée de poudre de zéolithe ou de mélanges de poudre de zéolithe avec au moins un agent liant.

3. Procédé selon la revendication 2, **caractérisé en ce que** l'agent liant est un agent liant anorganique, par exemple du ciment, du SiO₂ ou de la chaux ou un agent liant organique, par exemple un adhésif hydrorésistant ou un polymère, comme une résine phénolique ou un acrylate.

4. Etang de baignade ou piscine à traitement biologique avec un filtre à lit flottant contenant un substrat de filtrage à l'aide duquel on traite de l'eau pure (eau qui a déjà subi un traitement biologique et/ou chimique/physique) des eaux de baignade et qui est constitué de particules de substrat produites artificiellement,
caractérisé(e) en ce que
les particules de substrat comportent des particules porteuses qui sont dotées d'une enveloppe qui contient de la zéolithe synthétique sans phosphate, les particules de substrat ayant été conditionnées dans une solution nutritive par échange d'ions de telle sorte que les ions de sodium issus de la synthèse de la zéolithe soient remplacés par des ions d'ammonium, des ions de potassium et des ions de magnésium.

5. Etang de baignade ou piscine à traitement biologique selon la revendication 4, caractérisé(e) en ce que les particules porteuses des particules de substrat sont constituées de mousse de verre, d'argile, de graviers, de débris de pierre ou de matière plastique et en ce que l'enveloppe est constituée de poudre de zéolithe ou de mélanges de poudre de zéolithe avec au moins un agent liant.

6. Etang de baignade ou piscine à traitement biologique selon la revendication 5, caractérisé(e) en ce que l'agent liant est un agent liant anorganique, par exemple du ciment, du SiO₂ ou de la chaux ou un agent liant organique, par exemple un adhésif hydrorésistant ou un polymère, comme une résine phénolique ou un acrylate.
